# FASCICULE DE BREVET EUROPEEN

(11) **EP 2 356 443 B1**
(45) Date de publication et mention de la délivrance du brevet: **13.02.2013**
(21) Numéro de dépôt: 09801511.8
(22) Date de dépôt: 08.12.2009
(51) Int. Cl.: G01N 33/18, G01N 21/64, G01N 31/22

(54) **DOSAGE DU PLOMB**
BLEITESTVERFAHREN
LEAD ASSAY

(30) Priorité: 11.12.2008 FR 0806954
(43) Date de publication de la demande: 17.08.2011
(73) Titulaire: Centre National de la Recherche Scientifique - CNRS, 75016 Paris (FR)
(72) Inventeur: DELAIRE, Jacques, F-91120 Palaiseau (FR); LERAY, Isabelle, F-92130 Issy les Moulineaux (FR); LEFEVRE, Jean-Pierre, F-92800 Puteaux (FR); ZHAO, Liyun, Portland, OR 97201 (US); WU, Ting, Shanghai 200237 (CN)
(74) Mandataire: Derambure, Christian
(86) Numéro de dépôt international: PCT/FR2009/052450
(87) Numéro de publication internationale: WO 2010/067012

(56) Documents cités:
- US-A1- 2008 280 366
- CHANG, I-H; TULOCK, JJ; LIU, J; KIM, W-S; CANNON-JR, DM; LU,Y; BOHN, PW; SWEEDLER, JV; CROPEK, DM: "Miniaturized Lead Sensor Based on Lead-Specific DNAzyme in a Nanocapillary Interconnected Microfluidic Device" ENVIRON. SCI. TECHNOL., vol. 39, no. 10, 15 avril 2005 (2005-04-15), pages 3756-3761, XP002542230
- "Détection de cations polluants à l'aide de fluoroionophores sélectifs." "ÉPREUVE COMMUNE DE TIPE 2005", 2005, XP002542450
- TELTING-DIAZ, M; BAKKER, E: "Mass-Produced Ionophore-Based Fluorescent Microspheres for Trace Level Determination of Lead Ions" ANALYTICAL CHEMISTRY, vol. 74, no. 20, 15 octobre 2002 (2002-10-15), pages 5251-5256, XP002542452
- LU Y ET AL: "NEW HIGHLY SENSITIVE AND SELECTIVE CATALYTIC DNA BIOSENSORS FOR METAL IONS" BIOSENSORS AND BIOELECTRONICS, ELSEVIER BV, NL, vol. 18, no. 5/06, 1 mai 2003 (2003-05-01) , pages 529-540, XP001182221 ISSN: 0956-5663
- EMILIE DESTANDAU ET AL: "A novel microfluidic flow-injection analysis device with fluorescence detection for cation sensing. Application to potassium" 8 février 2007 (2007-02-08), ANALYTICAL AND BIOANALYTICAL CHEMISTRY, SPRINGER, BERLIN, DE, PAGE(S) 2627 - 2632 , XP019488814 ISSN: 1618-2650 cité dans la demande abrégé; figures 1,2

## Description

L'invention est relative au dosage du plomb.

Elle vise plus particulièrement un procédé de dosage du plomb dans de l'eau ou dans un échantillon solide et un dispositif de dosage du plomb pour la mise en oeuvre du procédé.

L'eau dont le plomb est à doser est typiquement une eau de rivière, une eau de surface, une eau de robinet, une eau de boisson ou autre. L'échantillon solide dont le plomb est à doser est typiquement de la peinture, le sol ou autre.

On connaît déjà des procédés et dispositifs de dosage par spectroscopie d'absorption ou émission atomique. Cette technique permet une bonne sélectivité et dans le cas du plomb une excellent sensibilité (par exemple 1 µg de plomb par litre, ou 1 ppb). Les dispositifs de mise en oeuvre sont typiquement destinés au laboratoire et sont à la fois coûteux et volumineux, de sorte qu'ils ne sont pas transportables. Par ailleurs, les opérations de dosage sont longues. Ces procédés et dispositifs ne sont donc pas adaptés au cas d'un dosage du plomb *in situ* au moyen d'un dispositif portable, de faible coût, rapide de mise en oeuvre mais de sensibilité suffisante au regard des normes en la matière.

On connaît également des procédés et dispositifs portables de dosage du plomb par voie électrochimique. Selon les réalisations, les dispositifs en question ont une sensibilité allant de 0,1 µg à 2 µg, ils nécessitent la mise en oeuvre d'une électrode au mercure, avec les problèmes inhérents, ou ils requièrent une durée d'analyse dépassant 3 à 5 minutes, ce qui les rend inadaptés à un usage *in situ* dans lequel les analyses doivent pouvoir être faites rapidement, ou encore ils sont onéreux et d'un emploi complexe.

« Lead and Mercury Sensing by Calixarene-Based Fluoroionophores Bearing Two or Four Dansyl Fluorophores », publié dans Chemistry - A European Journal, 2004, 10, pages 4480 à 4490, présente les propriétés photophysiques et complexantes de sondes fluorescentes dérivées de calixarènes et notamment la sélectivité et la sensibilité du composé Calix-DANS4 pour le plomb. Pour autant, il n'est pas décrit un dispositif complet de dosage du plomb adapté à un dosage *in situ.* Quant à la synthèse de ces calixarènes, elle a été décrite dans Chem. Commun. 2003, 996 (Métivier, Leray et Valeur). Le document XP-002542450 « Détection de cations polluants à l'aide de fluoroionophores sélectifs » (épreuve commune de TIPE 2005 - Partie D) vise la mise en oeuvre du composé Calix-DANS4 et il est couvert par les publications précédemment citées.

« A novel microfluidic flow-injection analysis device with fluorescence détection for cation sensing », publié dans Analytical & Bioanalytical Chemistry, 2007, 387:2627, pages 2627 à 2632, présente un dispositif microfluidique pour la détection des ions potassium par fluorimétrie. Un tel dispositif comprend deux entrées, une zone de mélange et une chambre de mesure de l'intensité de fluorescence à laquelle sont associés des moyens d'excitation optique et des moyens de recueil et d'analyse de l'intensité de fluorescence après excitation. Un tel dispositif est prévu en l'espèce pour le dosage des ions potassium mais non pour le plomb.

« Miniaturized Lead Sensor Based on Lead-Specific DNAzyme in a Nanocapillary Interconnected Microfluidinc Device », publié dans Environnemental Science & Technology, 2005, 10, pages 3756 à 3761, présente un procédé de dosage du plomb dans lequel, dans une première étape, on concentre le plomb et, dans une seconde étape, on mélange de l'eau de l'échantillon d'eau concentrée en plomb avec une sonde fluorescente sélective sensible au plomb, on excite par la lumière le mélange eau/sonde fluorescente ainsi réalisé et on recueille optiquement l'intensité de fluorescence du mélange eau/sonde fluorescente ainsi excité, et on détermine la dose en plomb de l'échantillon à doser, à partir de la fonction intensité de fluorescence / dose en plomb. Dans la réalisation décrite, la source d'excitation est un laser à argon ionisé ce qui interdit de mettre en oeuvre ce procédé au moyen d'un dispositif portatif. En outre, le procédé nécessite de mettre en oeuvre un microscope et deux alimentations haute tension, une pour l'électrophorèse capilalire (en cas de pré-séparation et pré-concentration) et une pour l'introduction de l'échantillon à travers la membranne nanoporeuse. Enfin, le procédé nécessite de mettre en oeuvre un DNAzyme spécifique du plomb avec son substrat.

On peut également citer dans l'état de la technique les documents suivants :
- « Chaotic mixer for microchannels », publié dans Science, vol. 295, 2002, pages 647 à 651 ;
- « Rate of access to the binding sites in organically modified silicates », publié dans Chem. Mater. 2002, 14, pages 2757 à 2766;
- « Mas-Produced lonophore-Based Fluorescent Microspheres for Trace Level Determination of Leads Ions », publié dans Anal. Chem. Vol. 74, n° 20, 15 octobre 2002, pages 5251 à 5256 qui repose sur une mesure de fluorescence dans une cuve à circulation qui n'est donc pas microfluidique ;
- « New highly sensitive and selective catalytic DNA biosensors for metal ions », publié dans Biosensors & Bioelectronics, Elsevier BV NL, vol. 18, n° 5/06, 1 er mai 2003, pages 529 à 540;
- « A novel microfluidic flow-injection analysis device with fluorescence détection for cation sensing. Application to potassium », publié par Anal Bional Chem, Spinger, 8 février 2007, pages 2627 à 2632 qui concerne un capteur microfluidique pour la détection du potassium ;
- US2008/0280366 qui décrit une méthode de détection du plomb par fluorescence de « clusters » (Pb₄Br₁₁)³⁻.

Il existe donc un besoin pour doser le plomb *in situ* au moyen d'un dispositif qui soit portable, de faible coût, rapide de mise en oeuvre, et de sensibilité suffisante au regard des normes en la matière. En effet, le plomb est un métal lourd très toxique, cause de différents effets très négatifs sur la santé humaine (troubles du comportement, épilepsie, saturnisme...). Le plomb peut se trouver dans l'eau (eau de rivière, de surface, du robinet, de boisson...) ou dans un support solide tel que de la peinture, le sol... Selon les normes européennes, la teneur limite en plomb autorisée dans l'eau de consommation humaine qui était dans le passé de 50 µg de plomb par litre (ou 50 ppb) a été limitée à partir de fin 2003 à 25 µg de plomb par litre (ou 25 ppb) et sera encore abaissée à 10 µg de plomb par litre (ou 10 ppb) à partir de fin 2013. Par conséquent, le dosage du plomb doit pouvoir être fait en de nombreuses circonstances, sur le terrain, dans des conditions parfois difficiles, rapidement et simplement, et enfin de façon sûre et précise. Les dispositifs mis en oeuvre doivent être compacts et transportables aisément. Ils doivent également pouvoir être fabriqués en grand nombre et ne pas générer de déchets toxiques.

L'invention vise à répondre à ce besoin.

A cet effet, selon un premier aspect, l'invention vise un procédé de dosage du plomb dans un échantillon d'eau telle qu'une eau de rivière, une eau de surface, une eau du robinet, une eau de boisson ou autre (échantillon d'eau originel), dans lequel on dispose d'un échantillon de l'eau dont on souhaite doser le plomb et dont au moins une partie substantielle de la matière colloïdale et organique est passée en solution (échantillon d'eau prétraitée), le procédé comportant les étapes successives suivantes :
- une première étape dans laquelle, partant d'un échantillon d'eau prétraitée à pH ajusté, on concentre le plomb pour obtenir un échantillon d'eau concentrée en plomb; et
- une seconde étape dans laquelle, partant de l'échantillon d'eau concentrée en plomb:
   o d'abord, on mélange de l'eau de l'échantillon d'eau concentrée en plomb avec une sonde fluorescente sélective sensible au plomb dont la fonction intensité de fluorescence / dose en plomb est connue,
   o puis on excite par la lumière le mélange eau/sonde fluorescente ainsi réalisé et on recueille optiquement l'intensité de fluorescence du mélange eau/sonde fluorescente ainsi excité,
   o enfin, à partir de l'intensité de fluorescence ainsi détectée, on détermine la dose en plomb de l'échantillon à doser, à partir de la fonction intensité de fluorescence /dose en plomb
caractérisé en ce que dans la première étape :
• dans une première sous-étape, on adsorbe le plomb en faisant passer l'échantillon d'eau • pré traitée à pH ajusté sur un adsorbant sélectif ne fixant pas les autres ions parasites;
• puis, dans une deuxième sous-étape, on procède à une désorption du plomb (échantillon d'eau concentrée en plomb) ;
• on élimine au moins pour partie les autres ions parasites pour obtenir un échantillon d'eau concentrée en plomb et substantiellement dépourvue des autres ions parasites..

On adsorbe le plomb en faisant passer l'échantillon d'eau pré traitée à pH ajusté sur des billes de silice fonctionnalisée; et on procède à une désorption par élution, l'éluant étant de l'eau acidifiée, pour obtenir un échantillon d'eau concentrée en plomb.

Selon les réalisations, dans la première étape, on concentre le plomb avec un facteur de concentration d'au moins 25, on élimine au moins pour partie les ions calcium et sodium, on élimine les ions parasites susceptibles d'interférer avec le plomb dans la seconde étape d'analyse par fluorescence, jusqu'à une concentration où ces ions parasites est telle que la mise en oeuvre de la seconde étape d'analyse par fluorescence est possible.

En outre, selon une réalisation, la première étape comporte une troisième sous-étape dans laquelle on procède à une filtration de l'eau d'élution obtenue par la désorption de la deuxième sous-étape, suivie d'une quatrième sous-étape dans laquelle le filtrat de la troisième sous-étape est additionné d'une solution de 3-chloropyridine dans l'acétonitrile, le pH final étant de 3.

Selon une réalisation, on part d'un échantillon d'eau originel et, dans une étape préliminaire, on met en solution au moins une partie substantielle de la matière colloïdale et organique, par une sous-étape d'acidification suivie d'une sous-étape de minéralisation (échantillon d'eau pré traitée). Dans ce cas, et selon une réalisation, l'étape préliminaire consiste en une acidification par addition d'acide nitrique concentré ultra-pur (69% HNO₃), à température ambiante, pendant une durée de l'ordre d'une heure, à un pH inférieur à 2. Selon une autre réalisation, l'étape préliminaire consiste en une sous-étape d'acidification à pH inférieur à 2, par addition d'acide nitrique et une sous-étape de minéralisation par addition de l'ordre de 2% d'eau oxygénée, à une température de l'ordre de 80°C, pendant une durée de l'ordre d'une heure.

Selon une réalisation, après l'étape préliminaire et avant la première étape, on porte le pH de l'échantillon d'eau pré traitée à une valeur de l'ordre de 10, notamment par ajout de soude.

Selon une autre caractéristique, pour la seconde étape, le pH est ajusté à 3 environ.

Selon une réalisation, la sonde fluorescente est le fluoroionophore Calix-DANS4, en solution dans un solvant qui est un mélange d'acétonitrile et d'eau à un pH de 3 dans le mélange, à une concentration de 2x10⁻⁶ M environ. Dans ce cas, dans l'échantillon d'eau concentrée en plomb, la concentration en calcium est inférieure à 100 mg/litre et la concentration en sodium inférieure à 11,5 mg/l, et ce compte tenu de la sélectivité de la sonde fluorescente.

Selon une réalisation, on réalise la seconde étape dans un dispositif microfluidique.

Selon une réalisation, on excite par la lumière le mélange eau/sonde fluorescente au moyen d'une diode luminescente et on recueille l'intensité de fluorescence du mélange eau/sonde fluorescente ainsi excité au moyen d'une fibre optique à laquelle est associé un photodétecteur..

Avec le procédé selon l'invention tel que décrit, on peut détecter le plomb jusqu'à une dose de 2 microgrammes par litre d'eau dont on souhaite doser le plomb.

Selon un deuxième aspect, l'invention vise un dispositif de dosage du plomb dans de l'eau telle qu'une eau de surface, une eau de boisson ou autre, pour la mise en oeuvre du procédé qui vient d'être décrit, qui comporte :
- un premier ensemble fonctionnel amont apte à permettre la réalisation de la première étape du procédé, et
- un second ensemble fonctionnel aval apte à permettre la réalisation de la seconde étape du procédé, ce second ensemble comportant:
   o un dispositif microfluidique ayant d'une part une entrée pour l'échantillon d'eau concentrée en plomb et d'autre part une entrée pour la sonde fluorescente, et, d'amont en aval et en communication fluidique, une zone de mélange de l'eau et de la sonde fluorescente, une chambre de mesure de l'intensité de fluorescence et une sortie des résidus,
   o des moyens lumineux d'excitation du mélange eau/sonde fluorescente et des moyens de recueil de l'intensité de fluorescence du mélange eau/sonde fluorescente ainsi excité, associés soit transversalement à la chambre de mesure, soit perpendiculairement les uns aux autres, l'excitation se faisant dans le plan de la chambre de mesure et le recueil de l'émission dans une direction perpendiculaire à cette chambre,
   o et des moyens de calcul programmés pour, à partir de l'intensité de fluorescence détectée, déterminer la dose en plomb de l'échantillon à doser, à partir de la fonction intensité de fluorescence / dose en plomb de la sonde fluorescente,
   dans lequel le premier ensemble comporte au moins un capillaire formant une micro-colonne de microbilles de silice fonctionnalisée, apte à être traversée par l'échantillon de l'eau prétraitée.

Selon une réalisation, la zone de mélange du dispositif microfluidique comporte des moyens de mélange actif ou passif, notamment des chevrons de mélange intégrés.

Selon une réalisation, la chambre de mesure de l'intensité de fluorescence du dispositif microfluidique est élargie et comporte des répartiteurs de flux intégrés.

Selon une réalisation, les moyens lumineux d'excitation du mélange eau/sonde fluorescente comprennent au moins une diode luminescente associée à au moins une fibre optique d'excitation et en ce que les moyens de recueil de l'intensité de fluorescence du mélange eau/sonde fluorescente comprennent au moins un photodétecteur associé à au moins une fibre optique de recueil.

Selon une réalisation adaptée au cas du procédé incluant une étape préliminaire, ainsi qu'il a été décrit plus haut, le dispositif comporte en outre, en amont du premier ensemble, un ensemble préliminaire apte à permettre la réalisation de cette étape préliminaire.

Selon une réalisation, le dispositif forme un ensemble au moins partiellement intégré, compact et portatif manuellement.

Selon un troisième aspect, l'invention vise un procédé de dosage du plomb dans un support solide, dans lequel :
- on extrait préalablement le plomb du support solide et on le solubilise dans de l'eau ne contenant pas de plomb ;
- puis on met en oeuvre le procédé de dosage du plomb dans de l'eau ainsi qu'il a été décrit plus haut.

On décrit maintenant plusieurs modes de réalisation de l'invention à l'aide des dessins dans lesquels :
- La figure 1 est une vue schématique en élévation d'un dispositif de dosage de plomb selon l'invention, comportant le premier ensemble fonctionnel amont et le second ensemble fonctionnel aval, ainsi qu'un ensemble préliminaire en amont du premier ensemble, ce dispositif étant adapté au cas où le procédé comporte l'étape préliminaire mentionnée précédemment ;
- La figure 2 est une vue schématique en élévation d'une partie du premier ensemble fonctionnel ;

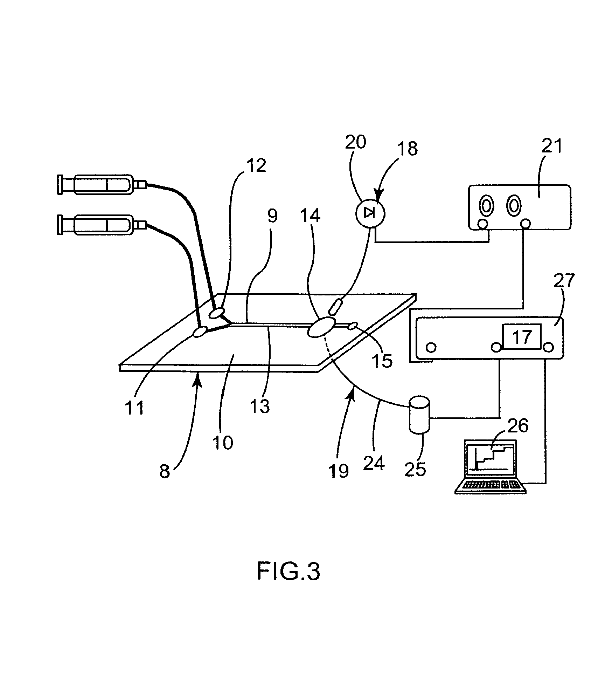

- La figure 3 est une vue schématique en perspective du second ensemble fonctionnel ;
- La figure 4 est une vue schématique en élévation du dispositif microfluidique du second ensemble fonctionnel ;
- La figure 5 est une vue schématique en élévation de la chambre de mesures du dispositif microfluidique du second ensemble fonctionnel ;
- La figure 6 est une courbe d'étalonnage du dispositif microfluidique du second ensemble fonctionnel.

On décrit maintenant le procédé de dosage du plomb se trouvant dans un échantillon d'eau que l'on dénomme, par convention, d'échantillon d'eau originel. Comme indiqué, l'eau est, selon les cas, de l'eau de rivière, de l'eau de surface, de l'eau du robinet, de l'eau de boisson ou toute autre eau dans laquelle on souhaite doser le plomb, notamment à des fins sanitaires.

Cet échantillon d'eau originel est prélevé *in situ,* et le procédé est mis en oeuvre *in situ* au moyen d'un dispositif de dosage 1. Le cas échéant, l'échantillon d'eau originel est transporté en un lieu où le dosage est effectué. Compte tenu du procédé mis en oeuvre, il est possible que le dispositif de dosage 1 soit portatif manuellement, formant un ensemble au moins partiellement intégré et compact, par exemple logé dans un carter 2. Ce dispositif de dosage 1 peut donc être transporté en tout lieu souhaité et en particulier *in situ* au lieu du prélèvement de l'échantillon originel à doser.

Partant de l'échantillon d'eau originel, et dans une étape préliminaire de pré traitement, on met en solution au moins une partie substantielle de la matière colloïdale et organique qu'il contient. A cet effet, on peut, selon une réalisation, procéder à une acidification par addition d'acide nitrique concentré ultra-pur (69% HNO₃), à température ambiante, pendant une durée de l'ordre d'une heure, à un pH inférieur à 2.

Selon une variante avec chauffage et ajout d'eau oxygénée, on réalise d'abord une sous-étape d'acidification à pH inférieur à 2, par addition d'acide nitrique concentré ultra-pur (69% HNO₃), puis une sous-étape de minéralisation par addition d'une quantité de 2% d'eau oxygénée, à une température de 80°C, pendant une durée de 1 heure.

Ainsi, au moins une partie substantielle de la matière colloïdale et organique de l'échantillon d'eau originel est passée en solution. L'échantillon d'eau ainsi obtenu après cette étape préliminaire de pré traitement est dénommé par convention échantillon d'eau prétraitée.

Il se peut que la matière colloïdale et organique ait été déjà solubilisée dans l'échantillon d'eau originel ou qu'il n'y en ait pas ou qu'il y en ait qui ne soit pas gênante pour la mise en oeuvre du procédé. Dans ce cas, l'étape préliminaire de pré traitement qui vient d'être décrite n'est pas réalisée. Par la suite de la description, on considère néanmoins, par convention, que l'étape préliminaire de pré traitement a été faite et que l'on dispose, en conséquence, d'un échantillon d'eau prétraitée.

Après cette étape préliminaire et/ou avant une première étape du procédé, on porte le pH de l'échantillon d'eau prétraitée à la valeur de 10, notamment par ajout d'une base telle que la soude, la potasse, l'ammoniaque.

On obtient ainsi un échantillon d'eau dénommé par convention échantillon d'eau prétraitée à pH ajusté.

La valeur de pH de 10 a été déterminée pour permettre une adsorption totale du plomb dans la première étape ultérieure du procédé.

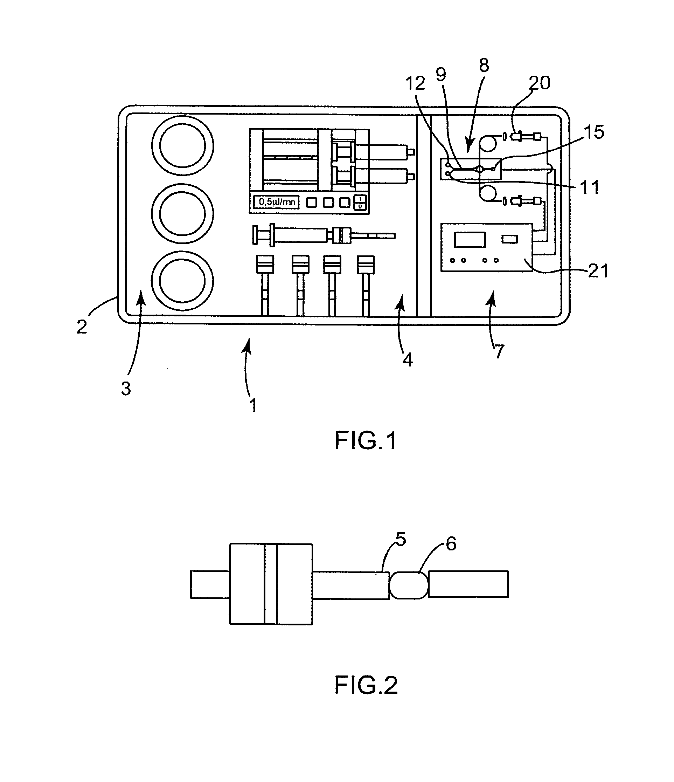

Le dispositif de dosage 1 comporte dans le carter 2, un ensemble préliminaire fonctionnel d'entrée 3, apte à permettre la réalisation de l'étape préliminaire de pré traitement suivie de l'ajustement du pH. Cet ensemble préliminaire fonctionnel d'entrée 3 (partie gauche de la figure 1) est placé fonctionnellement en amont d'un ensemble fonctionnel dénommé, par convention, premier ensemble fonctionnel de pré concentration 4.

En effet, le procédé comporte alors une première étape dans laquelle, partant de l'échantillon d'eau prétraitée à pH ajusté à une valeur voisine de 10, on concentre le plomb, en même temps qu'on élimine au moins pour partie les autres ions parasites. Par cette première étape, on obtient un échantillon d'eau dénommé par convention échantillon d'eau concentrée en plomb.

Pour ce faire, et dans une première sous-étape, on adsorbe les ions plomb Pb²⁺ de l'échantillon en faisant passer l'échantillon d'eau prétraitée à pH ajusté à une valeur voisine de 10 sur un adsorbant sélectif ne fixant pas les autres ions parasites.

Puis, dans une deuxième sous-étape, on procède à une désorption du plomb par élution à l'aide d'un tout petit volume d'une solution acide. Typiquement, pour 25 ml d'échantillon d'eau prétraitée ayant circulé sur la colonne, le volume de l'éluat est de 0,5mL, ce qui conduit à un facteur de concentration de 50. A pH 2, tout le plomb antérieurement adsorbé est désorbé par la suite.

Puis, dans une troisième sous-étape, on procède à une filtration de l'eau d'élution.

Enfin, dans une quatrième sous-étape, le filtrat est additionné d'une solution de 3-chloropyridine dans l'acétonitrile pour qu'à la fin, le pH final de la nouvelle solution soit de 3 dans un mélange d'acétonitrile (60% en volume) et d'eau (40% en volume). L'usage de ce tampon évite d'ajouter des ions alcalins ou alcalino-terreux susceptibles d'interférer avec le plomb par la suite.

A l'issue de cette quatrième sous-étape, suite à l'addition d'un petit volume d'eau, le facteur de concentration a été abaissé de 50 à 31,25, c'est-à-dire est d'au moins 25.

Ces sous-étapes sont réalisées dans le premier ensemble fonctionnel de pré concentration 4 (partie centrale de la figure 1).

A cette fin, et s'agissant de la première et de la deuxième sous-étapes, on fait passer l'échantillon d'eau prétraitée à pH ajusté à une valeur voisine de 10 sur des microbilles de silice fonctionnalisée avec des groupements 3-aminopropyl, Ref. Aldrich 36,425-8. Alternativement, on peut envisager l'utilisation de billes de silice nanoporeuse fonctionnalisée avec des ligands spécifiques des métaux lourds à détecter. Par conséquent, l'expression « microbilles de silice » doit être comprise de façon fonctionnelle et inclut toute structure équivalente.

Puis, on procède à une désorption par élution, l'éluant étant de l'eau pure acidifiée à pH voisin de 2, par exemple une solution aqueuse d'acide perchlorique. C'est ainsi qu'après filtration, ajustement du pH à 3 par addition de 3-chloropyridine dans un mélange acétonitrile/eau 60/40 en volume, on obtient ce que l'on a dénommé l'échantillon d'eau concentrée en plomb.

Dans une réalisation, et comme représenté schématiquement sur la figure 2, le premier ensemble de pré concentration 4 comporte au moins un capillaire 5, par exemple en polytétrafluoroéthylène (PTFE), rétréci par pincement à une extrémité, formant ou incluant une micro-colonne 6 de microbilles de silice fonctionnalisée. Ce capillaire 5 est apte à être traversé par l'échantillon d'eau prétraité à pH ajusté à une valeur voisine de 10. Le plomb, éventuellement d'autres métaux lourds, est adsorbé à la surface des microbilles de silice fonctionnalisée. En revanche, les ions calcium et sodium, souvent présents dans l'eau en grand excès, ne sont pas piégés par les microbilles de silice fonctionnalisée, de sorte que ces ions sont éliminés de l'éluat, une fois la désorption faite.

Pour éviter que la micro-colonne 6 de microbilles de silice fonctionnalisée ne soit colmatée par la matière colloïdale et organique présente dans l'échantillon d'eau originel, on a, comme mentionné précédemment, réalisé le pré traitement, dont la fonction est de solubiliser cette matière.

La filtration qui intervient après la désorption est réalisée au moyen, par exemple, d'un filtre en nitrate de cellulose ayant une taille de pores de 0,45 µm.

Le filtrat est additionné d'une solution de 3-chloropyridine dans l'acétonitrile pour qu'à la fin, le pH de la nouvelle solution soit de 3 dans un mélange d'acétonitrile (60% en volume) et d'eau (40% en volume). Ce mélange de solvants sera le même que le mélange utilisé pour dissoudre la sonde fluorescente dans la seconde étape d'analyse, cela afin d'éviter des effets thermiques dans le canal microfluidique.

La concentration en plomb réalisée dans de telles conditions est faite avec un facteur de concentration d'au moins 25 et plus spécialement égal ou de l'ordre de 30.

D'autre part, simultanément et par l'opération d'adsorption/désorption, on élimine au moins pour partie les ions calcium et sodium et, plus généralement, on élimine les ions parasites susceptibles d'interférer avec le plomb dans la seconde étape du procédé qui consiste en une analyse par fluorescence. Cette élimination est faite jusqu'à une concentration où ces ions parasites est telle que la mise en oeuvre de cette seconde étape est possible, c'est-à-dire qu'elle permet de déceler une intensité de fluorescence significative de la teneur en plomb effective. En effet, il a été constaté que l'ion calcium est un interférant du plomb à certaines concentrations mais non à d'autres, de sorte qu'il convient de limiter la concentration de l'ion calcium à une valeur en-dessous de laquelle il n'est pas interférant avec le plomb.

Bien entendu, les conditions opératoires de l'adsorption, de la désorption et de la filtration précédemment décrites sont optimisées, notamment en ce qui concerne le flux dans la colonne de microbilles 6 ou la concentration d'acide perchlorique dans la solution d'éluant.

Le procédé comporte alors la seconde étape précédemment mentionnée dans laquelle, partant de l'échantillon d'eau concentrée en plomb (ou analyte) et à un pH ajusté à 3 par addition préalable du tampon 3-chloropyridine, on procède à une analyse du plomb par fluorescence.

Cette seconde étape est réalisée dans un second ensemble fonctionnel d'analyse 7. Ce second ensemble 7 (partie droite de la figure 1) est placé fonctionnellement en aval du premier ensemble de pré concentration 4.

La seconde étape d'analyse est mise en oeuvre au moyen d'une sonde fluorescente sélective sensible au plomb et d'un dispositif microfluidique 8 faisant partie du second ensemble d'analyse 7.

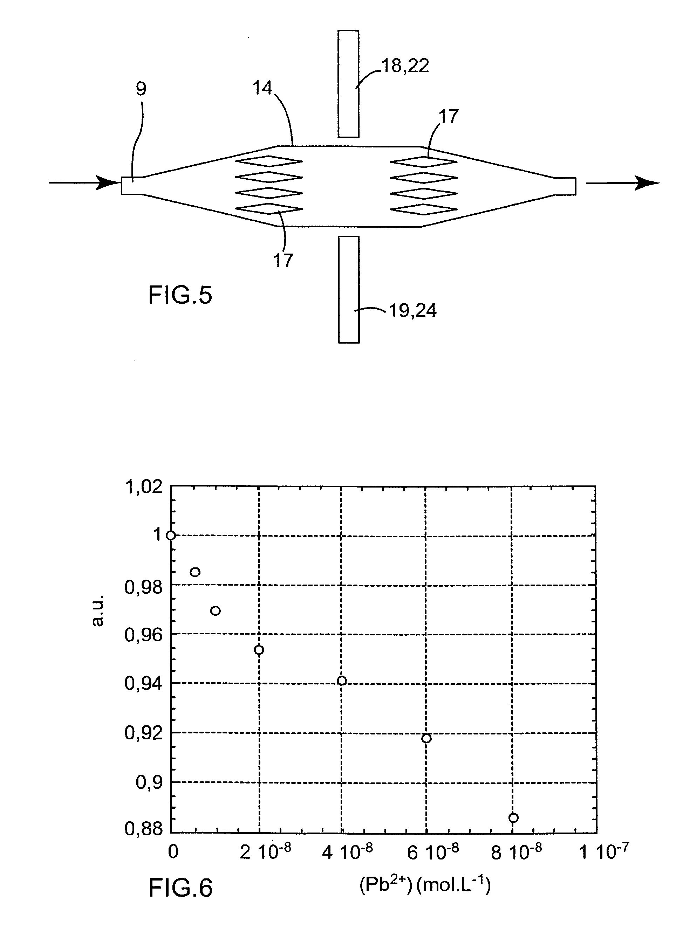

La fonction intensité de fluorescence / dose en plomb de la sonde fluorescente sélective sensible au plomb est connue ou a été établie par ailleurs. Cette fonction peut être illustrée par une courbe d'étalonnage telle que celle représentée sur la figure 6. On constate, en l'espèce, que cette courbe est proche d'une fonction linéaire décroissante. Ainsi, il est possible, connaissant cette fonction ou cette courbe, à partir d'une intensité de fluorescence donnée, parce que constatée, de déterminer sans risque d'erreur et avec précision, la dose en plomb correspondant à cette intensité de fluorescence. Cette fonction ou cette courbe peut être chargée dans des moyens de calcul programmés.

La seconde étape d'analyse comporte elle-même trois sous-étapes successives: une première sous-étape de mélange de l'échantillon d'eau concentrée en plomb avec la sonde fluorescente sélective sensible au plomb ; une deuxième sous-étape d'excitation par la lumière de ce mélange et de recueil optique de l'intensité de fluorescence de mélange ainsi excité ; et enfin, une troisième sous-étape de détermination de la dose en plomb de l'échantillon à doser, à partir de la fonction intensité de fluorescence / dose en plomb dont il a été question précédemment.

La sonde fluorescente est le fluoroionophore Calix-DANS4, en solution dans un solvant qui est un mélange d'acétonitrile (60% en volume) et d'eau (40% en volume) à un pH de 3 dans le mélange, et à une concentration de 2x10⁻⁶ M.

La formule structurelle du complexe Calix-DANS4/plomb est la suivante :

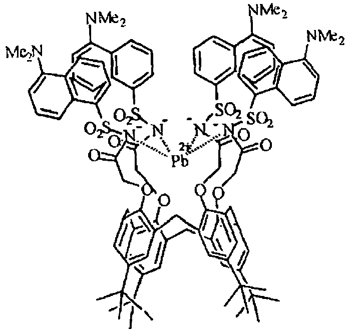

Dans ce cas considéré, il est possible, compte tenu de la sélectivité de la sonde fluorescente au regard du plomb que dans l'échantillon d'eau concentrée en plomb, la concentration en calcium soit ou puisse être inférieure à 100 mg/litre et la concentration en sodium soit, ou puisse être inférieure à 11.5 mg/l.

Comme il a été indiqué, il a été retenu un pH acide de 3. En effet, le pH d'utilisation de la sonde fluorescente est un paramètre important, car la complexation du plomb par les quatre azotes fixés sur les groupements dansyles est particulièrement sensible au pH. Un pH acide tend à ne pas permettre la complexation du plomb, les azotes étant protonnés. Un pH basique n'a pas cet effet, mais tend à ce que la variation de fluorescence constatée après excitation optique ne soit pas suffisante. Dès lors que, comme en l'espèce, l'excitation lumineuse est faite à une longueur d'onde de 365 nm et que la détection est faite à une longueur d'onde supérieure à 515 nm, il a été constaté qu'un pH de 3 était préférable pour assurer un dosage par fluorescence efficace.

Le dispositif microfluidique 8 se présente sous la forme d'un micro-conduit 9 en forme de Y moulé dans un matériau tel qu'un polymère siliconé tel le polydiméthylsiloxane (connu sous l'acronyme PDMS) fixé sur un substrat 10 en verre. En variante, le dispositif microfluidique 8 se présente sous la forme d'un micro-conduit 9 qui débute par une forme de Y et se poursuit par un trajet de longueur adaptée choisie pour réaliser une réaction de complexation totale.

Le micro-conduit 9 comporte une entrée 11 pour l'échantillon d'eau concentrée en plomb (analyte) et une entrée 12 pour la sonde fluorescente (réactif).

D'amont en aval et en communication fluidique, le micro-conduit 9 comporte une zone 13 allongée de mélange de l'eau et de la sonde fluorescente, une chambre 14 élargie de mesure de l'intensité de fluorescence et une sortie 15 des résidus.

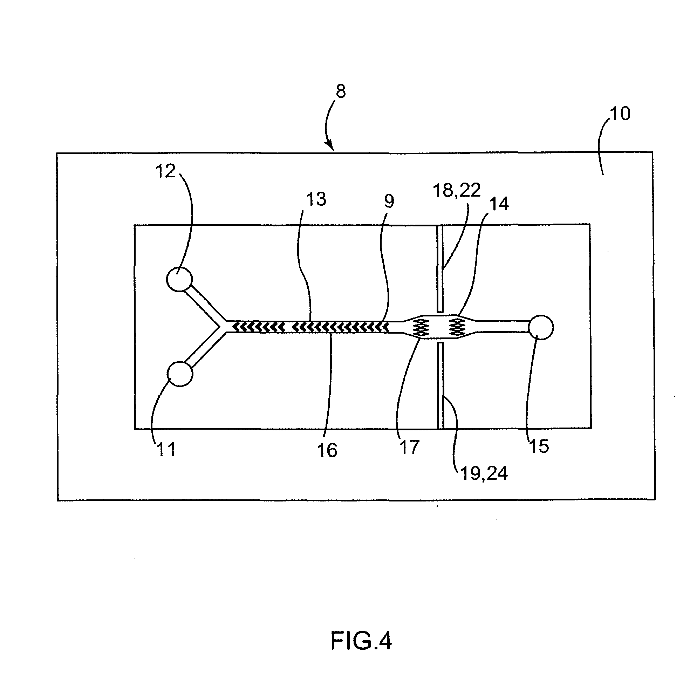

Dans la réalisation représentée sur la figure 4, il est prévu que la zone 13 de mélange soit pourvue de chevrons internes de mélange 16, aptes à assurer le mélange réactif/analyte de manière passive. Bien entendu, il peut être prévu au lieu et place une autre structure de mélange actif ou passif.

Dans la réalisation représentée sur la figure 5, il est prévu que la chambre 14 de mesure soit élargie et comporte un ou plusieurs répartiteurs de flux 17 sous forme de déflecteurs.

Au dispositif microfluidique 8 sont associés d'une part des moyens lumineux d'excitation 18 du mélange eau/sonde fluorescente et d'autre part des moyens optiques 19 de recueil de l'intensité de fluorescence du mélange eau/sonde fluorescente.

Ces moyens lumineux d'excitation 18 et moyens optiques 19 de recueil de l'intensité de fluorescence sont associés soit transversalement à la chambre de mesure 14, notamment en aval d'un répartiteur de flux 17 soit perpendiculairement les uns aux autres, l'excitation se faisant dans le plan de la chambre de mesure et le recueil de l'émission dans une direction perpendiculaire à cette chambre,

Selon une réalisation, les moyens lumineux d'excitation 18 comprennent au moins une diode luminescente 20, à laquelle sont associées une alimentation électrique 21 et une fibre optique d'excitation 22, le cas échéant une lentille 23.

Dans cette réalisation, la diode luminescente 20 est choisie pour émettre à 365 nm.

Selon une réalisation, les moyens optiques 19 de recueil de l'intensité de fluorescence comprennent une fibre optique de recueil conductrice 24, disposée orthogonalement par rapport à la fibre optique d'excitation 22, à laquelle est associé un photodétecteur 25, devant lequel est disposé un filtre optique passe-haut 28 (λ> 515 nm).

Au dispositif microfluidique 8 sont également associés des moyens 26 de calcul programmés pour, à partir de l'intensité de fluorescence détectée et collectée, déterminer la dose en plomb de l'échantillon à doser, à partir de la fonction intensité de fluorescence / dose en plomb de la sonde fluorescente, chargée dans les moyens 26. Ces moyens 26 de calcul, tels qu'un ordinateur, comportent des moyens de visualisation, stockage, impression de données.

En outre, un amplificateur à détection synchrone 27 permet de s'affranchir du bruit de fond.

Il est entendu que les étapes du procédé précédemment décrit peuvent être intégrées de façon plus ou moins grande, ce qui implique alors d'intégrer de façon plus ou moins élevée le dispositif microfluidique 8 dans le dispositif de dosage 1. Ainsi, le dispositif microfluidique 8 pourrait être conçu avec une seule entrée pour l'échantillon à doser, tandis qu'une réserve de sonde fluorescente serait intégrée au dispositif de dosage 1, le dispositif microfluidique 8 étant pourvu de valves, vannes... appropriées.

Avec le procédé selon l'invention tel que décrit, on peut détecter le plomb jusqu'à une dose de 2 µg par litre d'eau dont on souhaite doser le plomb, ce qui est bien mieux que le seuil requis pour les eaux de boisson par les normes pour 2013, soit 10 µg par litre d'eau.

Le procédé a par ailleurs l'avantage d'être sélectif du plomb. En effet, le dosage du plomb peut être effectué malgré la présence dans l'eau de calcium et de sodium, jusqu'à des concentrations de 11,5 mg par litre d'eau pour celui-ci et de 100 mg par litre d'eau pour celui-là.

Même dans l'hypothèse où d'autres ions de métaux lourds présents à l'état de traces, comme le plomb, étaient préconcentrés après passage dans la micro-colonne, ils ne seraient pas détectés au cours de la seconde étape, compte tenu de la sélectivité de la sonde fluorescente pour le plomb.

Selon un développement de l'invention, le procédé de dosage du plomb est destiné à un support solide, tel que de la peinture, le sol ou autre.

Dans ce cas, on extrait préalablement le plomb d'un échantillon du support solide et on le solubilise dans de l'eau ne contenant pas de plomb. Puis on met en oeuvre le procédé de dosage du plomb dans de l'eau ainsi qu'il a été décrit précédemment.

## Revendications

1. Procédé de dosage du plomb dans un échantillon d'eau telle qu'une eau de rivière, une eau de surface, une eau du robinet, une eau de boisson ou autre (échantillon d'eau originel), dans lequel on dispose d'un échantillon de l'eau dont on souhaite doser le plomb et dont au moins une partie substantielle de la matière colloïdale et organique est passée en solution (échantillon d'eau prétraitée), le procédé comportant les étapes successives suivantes :
• une première étape dans laquelle, partant d'un échantillon d'eau prétraitée à pH ajusté, on concentre le plomb pour obtenir un échantillon d'eau concentrée en plomb; et
• une seconde étape dans laquelle, partant de l'échantillon d'eau concentrée en plomb:
o d'abord, on mélange de l'eau de l'échantillon d'eau concentrée en plomb avec une sonde fluorescente sélective sensible au plomb dont la fonction intensité de fluorescence / dose en plomb est connue,
o puis on excite par la lumière le mélange eau/sonde fluorescente ainsi réalisé et on recueille optiquement l'intensité de fluorescence du mélange eau/sonde fluorescente ainsi excité,
o enfin, à partir de l'intensité de fluorescence ainsi détectée, on détermine la dose en plomb de l'échantillon à doser, à partir de la fonction intensité de fluorescence / dose en plomb
**caractérisé en ce que** dans la première étape :
■ dans une première sous-étape, on adsorbe le plomb en faisant passer l'échantillon d'eau pré traitée à pH ajusté sur un adsorbant sélectif ne fixant pas les autres ions parasites;
■ puis, dans une deuxième sous-étape, on procède à une désorption du plomb (échantillon d'eau concentrée en plomb) ;
■ on élimine au moins pour partie les autres ions parasites pour obtenir un échantillon d'eau concentrée en plomb et substantiellement dépourvue des autres ions parasites.

2. Procédé selon la revendication 1, **caractérisé en ce que** dans la première étape :
■ on adsorbe le plomb en faisant passer l'échantillon d'eau pré traitée à pH ajusté sur des billes de silice fonctionnalisée ;
■ et on procède à une désorption par élution, l'éluant étant de l'eau acidifiée, pour obtenir un échantillon d'eau concentrée en plomb.

3. Procédé selon l'une quelconque des revendications 1 et 2, **caractérisé en ce que** dans la première étape, on concentre le plomb avec un facteur ce concentration d'au moins 25.

4. Procédé selon l'une quelconque des revendications 1 à 3, **caractérisé en ce que** dans la première étape, on élimine au moins pour partie les ions calcium et sodium.

5. Procédé selon l'une quelconque des revendications 1 à 4, **caractérisé en ce que** dans la première étape, on élimine les ions parasites susceptibles d'interférer avec le plomb dans la seconde étape d'analyse par fluorescence, jusqu'à une concentration où ces ions parasites est telle que la mise en oeuvre de la seconde étape d'analyse par fluorescence est possible.

6. Procédé selon l'une quelconque des revendications 1 à 4, **caractérisé en ce que** la première étape comporte une troisième sous-étape dans laquelle on procède à une filtration de l'eau d'élution obtenue par la désorption de la deuxième sous-étape.

7. Procédé selon la revendication 6, **caractérisé en ce que** la troisième sous-étape est suivie d'une quatrième sous-étape dans laquelle le filtrat obtenu de la troisième sous-étape est additionné d'une solution de 3-chloropyridine dans l'acétonitrile, le pH final étant de 3.

8. Procédé selon l'une quelconque des revendications 1 à 7, **caractérisé en ce que** l'on part d'un échantillon d'eau originel et, dans une étape préliminaire, on met en solution au moins une partie substantielle de la matière colloïdale et organique.

9. Procédé selon la revendication 8, **caractérisé par** une étape préliminaire consistant en une acidification par addition d'acide nitrique concentré ultra-pur (69% HNO₃), à température ambiante, pendant une durée de l'ordre d'une heure, à un pH inférieur à 2.

10. Procédé selon la revendication 8, **caractérisé par** une étape préliminaire consistant en une sous-étape d'acidification à pH inférieur à 2, par addition d'acide nitrique et une sous-étape de minéralisation par addition de l'ordre de 2% d'eau oxygénée, à une température de l'ordre de 80°C, pendant une durée de l'ordre d'une heure.

11. Procédé selon l'une quelconque des revendications 1 à 10, **caractérisé en ce qu'**après l'étape préliminaire et/ou avant la première étape, on porte le pH de l'échantillon d'eau pré traitée à une valeur de l'ordre de 10, en particulier par ajout d'une base telle que la soude, la potasse, l'ammoniaque.

12. Procédé selon l'une quelconque des revendications 1 à 11, caractérisé en ce pour la seconde étape, le pH est ajusté à 3 environ.

13. Procédé selon l'une quelconque des revendications 1 à 12, caractérisé en ce la sonde fluorescente est le fluoroionophore Calix-DANS4, en solution dans un solvant qui est un mélange d'acétonitrile et d'eau à un pH de 3 dans le mélange.

14. Procédé selon l'une quelconque des revendications 1 à 13, **caractérisé en ce que** la sonde fluorescente est à une concentration de 2x10⁻⁶ M environ.

15. Procédé selon l'une quelconque des revendications 1 à 14, caractérisé en ce dans l'échantillon d'eau concentrée en plomb, la concentration en calcium est inférieure à 100 mg/litre et la concentration en sodium inférieure à 11,5 mg/l.

16. Procédé selon l'une quelconque des revendications 1 à 15, **caractérisé en ce qu'**on détecte le plomb jusqu'à une dose de 2 µg par litre d'eau dont on souhaite doser le plomb.

17. Dispositif de dosage du plomb dans de l'eau telle qu'une eau de surface, une eau de boisson ou autre, pour la mise en oeuvre du procédé selon l'une quelconque des revendications 1 à 16, qui comporte :
■ un premier ensemble fonctionnel amont apte à permettre la réalisation de la première étape du procédé, et
■ un second ensemble fonctionnel aval apte à permettre la réalisation de la seconde étape du procédé, ce second ensemble comportant:
o un dispositif microfluidique ayant d'une part une entrée pour l'échantillon d'eau concentrée en plomb et d'autre part une entrée pour la sonde fluorescente, et, d'amont en aval et en communication fluidique, une zone de mélange de l'eau et de la sonde fluorescente, une chambre de mesure de l'intensité de fluorescence et une sortie des résidus,
o des moyens lumineux d'excitation du mélange eau/sonde fluorescente et des moyens de recueil de l'intensité de fluorescence du mélange eau/sonde fluorescente ainsi excité, associés soit transversalement à la chambre de mesure, soit perpendiculairement les uns aux autres, l'excitation se faisant dans le plan de la chambre de mesure et le recueil de l'émission dans une direction perpendiculaire à cette chambre,
○ et des moyens de calcul programmés pour, à partir de l'intensité de fluorescence détectée, déterminer la dose en plomb de l'échantillon à doser, à partir de la fonction intensité de fluorescence / dose en plomb de la sonde fluorescente,
**caractérisé en ce que** le premier ensemble comporte au moins un capillaire formant une micro-colonne de microbilles de silice fonctionnalisée, apte à être traversée par l'échantillon de l'eau prétraitée.

18. Dispositif selon la revendication 17, **caractérisé en ce que** la zone de mélange du dispositif microfluidique comporte des moyens de mélange actif ou passif, notamment des chevrons de mélange intégrés.

19. Dispositif selon l'une quelconque des revendications 17 et 18, **caractérisé en ce que** la chambre de mesure de l'intensité de fluorescence du dispositif microfluidique élargie et comporte des répartiteurs de flux intégrés.

20. Dispositif selon l'une quelconque des revendications 17 à 19, **caractérisé en ce que** les moyens lumineux d'excitation du mélange eau/sonde fluorescente comprennent au moins une diode luminescente associée à au moins une fibre optique d'excitation et **en ce que** les moyens de recueil de l'intensité de fluorescence du mélange eau/sonde fluorescente comprennent au moins un photodétecteur associé à au moins une fibre optique de recueil.

21. Dispositif selon l'une quelconque des revendications 17 à 20 en ce qu'il dépend du procédé selon l'une quelconque des revendications 8 à 16, **caractérisé en ce qu'**il comporte en outre, en amont du premier ensemble, un ensemble préliminaire apte à permettre la réalisation de l'étape préliminaire du procédé.

22. Procédé de dosage du plomb dans un support solide, dans lequel :
- on extrait préalablement le plomb du support solide et on le solubilise dans de l'eau ne contenant pas de plomb ;
- puis on met en oeuvre le procédé de dosage du plomb dans de l'eau selon l'une quelconque des revendications 1 à 16.

## Claims

1. A method for determining the lead content in a sample of water such as river water, surface water, tap water, drinking water or any other water (original water sample), wherein is provided a sample of water, the lead content of which is to be determined, and of which at least a substantial portion of the colloidal and organic material is dissolved (pre-treated water sample), with the method including the following successive steps:
• a first step wherein, from a sample of pre-treated water with an adjusted pH, lead is concentrated in order to obtain a lead-concentrated water sample; and
• a second step, wherein, from the lead-concentrated water sample:
o first, water from the lead-concentrated water sample is mixed with a selective fluorescent probe responsive to lead, the fluorescent brightness/lead content function of which is known,
o then the water/fluorescent probe mixture thus obtained is excited using a light-beam and the fluorescent brightness of the thus excited water/fluorescent probe mixture is optically collected,
o eventually, from the thus detected fluorescent brightness, the lead content of the sample to be analyzed is determined, from the fluorescent brightness/lead content function
**characterized in that**, during the first step:
• during the first sub-step, lead is adsorbed by passing the sample of pre-treated water with an adjusted pH through a selective adsorbent, which does not fix the other unwanted ions;
• then during a second sub-step, lead desorption is carried out (sample of lead-concentrated water) ;
• the other unwanted ions are at least partially eliminated in order to obtain a sample of lead-concentrated water substantially free of other unwanted ions.

2. A method according to claim 1, **characterized in that**, during the first step:
• lead is adsorbed by passing the pre-treated water sample with an adjusted pH on functionalized silica beads;
• and elution desorption is carried out, with the eluent being acidified water, in order to obtain a lead-concentrated water sample.

3. A method according to any one of claims 1 and 2, **characterized in that**, during the first step, lead is concentrated with a concentration factor of at least 25.

4. A method according to any one of claims 1 to 3, **characterized in that**, during the first step, calcium and sodium ions are at least partially eliminated.

5. A method according to any one of claims 1 to 4, **characterized in that**, during the first step, the unwanted ions liable to interfere with lead during the second step of fluorescence analysis are eliminated, up to a concentration wherein such unwanted ions is such that the implementation of the second step of fluorescence analysis is possible.

6. A method according to any one of claims 1 to 4, **characterized in that** the first step includes a third sub-step wherein a filtration of the elution water obtained by the desorption of the second sub-step is carried out.

7. A method according to claim 6, **characterized in that** the third sub-step is followed by a fourth sub-step wherein the filtrate obtained from the third sub-step receives a solution of 3-chloropyridine in acetonitrile, with the final pH being 3.

8. A method according to any one of claims 1 to 7, **characterized in that** the starting element is an original water sample, and during a preliminary step, at least a substantial portion of the colloidal and organic material is dissolved.

9. A method according to claim 8, **characterized by** a preliminary step consisting of an acidification by addition of ultra-pure (69% HNO₃) concentrated nitric acid, at room temperature, for a duration of approximately one hour, at a pH of less than 2.

10. A method according to claim 8, **characterized by** a preliminary step consisting of a sub-step of acidification to a pH of less than 2, by adding nitric acid, and a sub-step of mineralization by adding about 2% of hydrogen peroxide, at a temperature of approximately 80°C, for a duration of about one hour.

11. A method according to any one of claims 1 to 10, **characterized in that**, after the preliminary step and/or prior to the first step, the pH of the pre-treated water sample is changed for a value of 10, more particularly by adding a base such as soda, potash, ammonia.

12. A method according to any one of claims 1 to 11, **characterized in that**, during the second step, the pH is adjusted to approximately 3.

13. A method according to any one of claims 1 to 12, **characterized in that** the fluorescent probe is Calix-DANS4 fluoroionophore, dissolved in a solvent which is a mixture of acetonitrile and water, with a pH of 3 in the mixture.

14. A method according to any one of claims 1 to 13, **characterized in that** the fluorescent probe has a concentration of approximately 2x10 ⁶M.

15. A method according to any one of claims 1 to 14, **characterized in that**, in the lead-concentrated water sample, the calcium content is less than 100mg/l and the sodium content is less than 11.5mg/l.

16. A method according to any one of claims 1 to 15, **characterized in that** lead is detected up to a content of 2µg per litre of water the lead content of which is to be determined.

17. A device for determining the lead content in water such as surface water, drinking water or any other water, for implementing the method according to any one of claims 1 to 16, which includes:
a first upstream functional assembly able to enable the execution of the first step of the method, and
• a second downstream functional assembly able to enable the execution of the second step of the method, with the second assembly including:
○ a micro-fluid device with, on the one hand, an inlet for the sample of lead-concentrated water, and on the other hand an inlet for the fluorescent probe, and from upstream to downstream and in fluid communication, a water and fluorescent probe mixing area, a fluorescent brightness measuring chamber and a waste outlet,
○ light-beam means for exciting the water/fluorescent probe mixture and means for collecting the fluorescent brightness of the thus excited water/fluorescent probe mixture, associated either transversally with the measuring chamber, or perpendicularly with one another, with the excitation occurring in the plane of the measuring chamber and the collection of the emission in a direction perpendicular to the chamber,
○ and calculation means programmed for, from the detected fluorescent brightness, determining the lead content of the sample to be analyzed, from the fluorescent brightness/lead content function of the fluorescent probe,
**characterized in that** the first assembly includes at least one capillary tube forming a micro-column of functionalized silica micro-beads, able to be gone through by the sample of pre-treated water.

18. A device according to claim 17, **characterized in that** the micro-fluid device mixing area includes active or passive mixing means, more particularly integrated mixing chevrons.

19. A device according to any one of claims 17 and 18, **characterized in that** the micro-fluid device fluorescent brightness measuring chamber is widened and includes integrated flux distributors.

20. A device according to any one of claims 17 to 19, **characterized in that** the light-beam means for exciting the water/fluorescent probe mixture includes at least a light-emitting diode associated with at least one exciting optical fibre and **in that** the means for collecting the fluorescent brightness of the water/fluorescent probe mixture includes at least one photo-detector associated with at least one collecting optical fibre.

21. A device according to any one of claims 17 to 20, **characterized in that** it depends on the method according to any one of claims 8 to 16, **characterized in that** it further includes, upstream from the first assembly, a preliminary assembly able to enable the execution of the preliminary step of the method.

22. A method for determining the lead content in a solid support, wherein:
- lead is previously extracted from the solid support and is solubilised in water containing no lead;
- the method for determining the lead content in water according to any one of claims 1 to 16 is implemented.

## Patentansprüche

1. Dosierungsverfahren des Bleis in einer Wasserprobe, wie z. B. Flusswasser, Oberflächenwasser, Leitungswasser, Trinkwasser oder Sonstiges (Probe ursprünglichen Wassers), bei dem eine Probe des Wassers, dessen Blei dosiert werden soll und von dem wenigstens ein wesentlicher Teil des kolloidalen und organischen Materials in Lösung übergegangen ist (Probe vorbehandelten Wassers) angeordnet wird, wobei das Verfahren die folgenden sukzessiven Stufen umfasst:
* eine erste Stufe, in der ausgehend von einer Probe vorbehandelten Wassers mit einem angepassten ph-Wert das Blei konzentriert wird, um eine Wasserprobe mit Bleikonzentration zu erhalten; und
* eine zweite Stufe, in der ausgehend von der Wasserprobe mit Bleikonzentration:
- zunächst das Wasser der Wasserprobe mit Bleikonzentration mit einer für Blei sensiblen, selektiven, fluoreszierenden Sonde, deren intensivierte Fluoreszenz-Funktion / Bleidosis bekannt ist, vermischt wird,
- dann durch das Licht die auf diese Weise realisierte Mischung Wasser / Fluoreszenzsonde erregt wird und optisch die Intensität der Fluoreszenz der auf diese Weise erregte Mischung Wasser / Fluoreszenzsonde aufgefangen wird,
- dann ausgehend von der auf diese Weise festgestellte Intensität der Fluoreszenz die Bleidosis der zu dosierenden Probe ausgehend von der Intensitätsfunktion der Fluoreszenz / Bleidosis bestimmt wird
**dadurch gekennzeichnet, dass** in der ersten Stufe:
* In einer ersten Unterstufe das Blei adsorbiert wird, indem die Probe des vorbehandelten Wassers mit angepasstem ph-Wert auf einem selektiven Adsorptionsmittel vorbehandelt wird, das die anderen parasitären Ionen nicht fixiert;
* Dann, in einer zweiten Stufe, eine Desorption des Bleis vorgenommen wird (Wasserprobe mit Bleikonzentration);
* Wenigstens zum Teil die anderen parasitären Ionen eliminiert werden, um eine Wasserprobe mit Bleikonzentration zu erhalten, die im Wesentlichen frei von den anderen parasitären Ionen ist.

2. Verfahren gemäß Anspruch 1, **dadurch gekennzeichnet, dass** in der ersten Stufe:
* das Blei adsorbiert wird, indem die Probe des vorbehandelten Wassers mit angepasstem ph-Wert über funktionalisierte Siliziumkugeln geleitet wird;
* und eine Desorption per Elution vorgenommen wird, wobei das Extraktionsmittel das angesäuerte Wasser ist, um eine Wasserprobe mit Bleikonzentration zu erhalten.

3. Verfahren gemäß Anspruch 1 und 2, **dadurch gekennzeichnet, dass** in der ersten Stufe das Blei mit einem Konzentrationsfaktor von mindestens 25 konzentriert wird.

4. Verfahren gemäß Anspruch 1 bis 3, **dadurch gekennzeichnet, dass** in der ersten Stufe die Kalzium- und Natriumionen wenigstens zum Teil eliminiert werden.

5. Verfahren gemäß Anspruch 1 bis 4, **dadurch gekennzeichnet, dass** in der ersten Stufe die parasitären Ionen, die geeignet sind, mit dem Blei in der zweiten Analysestufe per Fluoreszenz zu interferieren, bis zu einer Konzentration zu eliminieren, in der diese parasitären Ionen derart sind, dass die Umsetzung der zweiten Analysestufe per Fluoreszenz möglich ist.

6. Verfahren gemäß Anspruch 1 bis 4, **dadurch gekennzeichnet, dass** das erste Verfahren eine dritte Unterstufe umfasst, in der eine Filterung des Elutionswassers durchgeführt wird, das durch die Desorption der zweiten Unterstufe erhalten wurde.

7. Verfahren gemäß Anspruch 6, **dadurch gekennzeichnet, dass** die dritte Unterstufe von einer vierten Unterstufe gefolgt wird, in der das aus der dritten Unterstufe erhaltene Filtrat zu einer Lösung aus 3-Chlorpyridin in dem Acetonitril hinzugefügt wird, wobei der endgültige ph-Wert 3 ist.

8. Verfahren gemäß Anspruch 1 bis 7, **dadurch gekennzeichnet, dass** von einer Probe ursprünglichen Wassers ausgegangen wird und in einer einleitenden Stufe wenigstens ein wesentlicher Teil des kolloidalen und organischen Materials in eine Lösung gegeben wird.

9. Verfahren gemäß Anspruch 8, **gekennzeichnet durch** eine einleitende Stufe, die aus einer Ansäuerung per Hinzufügen von konzentrierter, ultrareiner Salpetersäure (69 % HNO₃) bei Raumtemperatur über eine Dauer in der Größenordnung von einer Stunde bei einem ph-Wert von weniger als 2 besteht.

10. Verfahren gemäß Anspruch 8, **gekennzeichnet durch** eine einleitende Stufe, die aus einer Ansäuerungs-Unterstufe mit einem ph-Wert von weniger als 2 durch Hinzufügen von Salpetersäure und einer Mineralisierungs-Unterstufe **durch** Hinzufügen in der Größenordnung von 2 % Wasserstoffperoxid bei einer Temperatur in der Größenordnung von 80°C über eine Dauer in der Grö-βenordnung von einer Stunde besteht.

11. Verfahren gemäß Anspruch 1 bis 10, **dadurch gekennzeichnet, dass** nach der einleitenden Stufe und /oder vor der ersten Stufe der ph-Wert der Probe des vorbehandelten Wassers auf einen Wert in der Größenordnung von 10 insbesondere durch das Hinzufügen einer Basis, wie z. B. Natron, Ätzkali, Ammoniak gebracht wird.

12. Verfahren gemäß Anspruch 1 bis 11, **dadurch gekennzeichnet, dass** der ph-Wert für die zweite Stufe auf rund 3 gebracht wird.

13. Verfahren gemäß Anspruch 1 bis 12, **dadurch gekennzeichnet, dass** die fluoreszierende Sonde das Fluorionophor Calix-DANS4 in Lösung in einem Lösungsmittel ist, das eine Mischung aus Acetonitril und Wasser mit einem ph-Wert von 3 in der Mischung ist.

14. Verfahren gemäß Anspruch 1 bis 13, **dadurch gekennzeichnet, dass** die fluoreszierende Sonde eine Konzentration von rund 2x10⁻⁶ M ist.

15. Verfahren gemäß Anspruch 1 bis 14, **dadurch gekennzeichnet, dass** in der Wasserprobe mit Bleikonzentration die Kalziumkonzentration unter 100 mg / Liter und die Natriumkonzentration unter 11,5 mg/l liegt.

16. Verfahren gemäß Anspruch 1 bis 15, **dadurch gekennzeichnet, dass** das Blei bis zu einer Dosis von 2 µg pro Liter Wasser festgestellt wird, dessen Bleigehalt dosiert werden soll.

17. Dosierungsvorrichtung des Bleis in Wasser, wie z. B. Oberflächenwasser, Trinkwasser oder Sonstiges für die Umsetzung des Verfahrens gemäß Anspruch 1 bis 16, die umfasst:
* eine erste vorgeschaltete funktionale Struktur, die geeignet ist, die Realisierung der ersten Stufe des Verfahrens zu erlauben, und
* eine zweite nachgeschaltete funktionale Struktur, die geeignet ist, die Realisierung der zweiten Stufe des Verfahrens zu erlauben, wobei diese zweite Struktur umfasst:
- eine mikrofluidische Vorrichtung, die einerseits einen Einlass für die Wasserprobe mit Bleikonzentration und andererseits einen Einlass für die Fluoreszenzsonde und von oben nach unten und in fluidischer Kommunikation eine Mischzone des Wassers und der fluoreszierenden Sonde, eine Messkammer der Fluoreszintensität und einen Auslass der Rückstände hat,
- leuchtende Erregungsmittel der Mischung Wasser / fluoreszierende Sonde und Auffangmittel der Fluoreszenzintensität der auf diese Weise erregten Mischung Wasser / fluoreszierende Sonde, die entweder transversal zur Messkammer oder lotrecht zueinander zugeordnet sind, wobei die Erregung in der Ebene der Messkammer und die Aufnahme der Ausgabe in einer zu dieser Kammer lotrechten Richtung erfolgen,
- und ausgehend von der Intensität der festgestellten Fluoreszenz programmierte Berechnungsmittel zur Bestimmung der Bleidosis der zu dosierenden Probe ausgehend von der Intensitätsfunktion der Fluoreszenz / Bleidosis in der fluoreszierenden Sonde,
**dadurch gekennzeichnet, dass** die erste Struktur wenigsten ein Haargefäß umfasst, das eine funktionalisierte Mikrosäule aus Silizium-Mikrokugeln umfasst, die geeignet ist, von der Probe vorbehandelten Wassers durchquert zu werden.

18. Vorrichtung gemäß Anspruch 17, **dadurch gekennzeichnet, dass** der Mischbereich der mikrofluidischen Vorrichtung Mittel zur aktiven oder passiven Mischung, insbesondere integrierte Misch-Spitzköper umfasst.

19. Vorrichtung gemäß Anspruch 17 und 18, **dadurch gekennzeichnet, dass** die Messkammer der Fluoreszenzintensität der mikrofluidischen Vorrichtung integrierte Flussverteiler erweitert und umfasst.

20. Vorrichtung gemäß Anspruch 17 bis 19, **dadurch gekennzeichnet, dass** die leuchtenden Erregungsmittel der Mischung Wasser / fluoreszierende Sonde wenigstens eine Leuchtdiode umfassen, die wenigstens einer Erregungsglasfaser zugeordnet ist und dass die Auffangmittel der Fluoreszenzintensität der Mischung Wasser /fluoreszierende Sonde wenigstens einen Fotodetektor umfassen, der wenigstens einer Auffang-Glasfaser zugeordnet ist.

21. Vorrichtung gemäß Anspruch 17 bis 20 als sie von dem Verfahren gemäß Anspruch 8 bis 16 abhängt, **dadurch gekennzeichnet, dass** sie darüber hinaus vor der ersten Struktur vorgeschaltet eine einleitende Struktur umfasst, die geeignet ist, die Realisierung der einleitenden Stufe des Verfahrens zu realisieren.

22. Dosierungsverfahren des Bleis in einem festen Träger, bei dem:
- Zuvor das Blei des festen Trägers extrahiert und es wird in dem Wasser gelöst wird, das kein Blei enthält;
- Dann das Dosierungsverfahren des Bleis in dem Wasser gemäß Anspruch 1 bis 16 umgesetzt wird.
